# EUROPEAN PATENT APPLICATION

(11) **EP 4 740 921 A1**
(43) Date of publication of application: **13.05.2026**
(21) Application number: 24877060.4
(22) Date of filing: 01.10.2024
(51) Int. Cl.: A61F 13/51, A61F 13/496, A61F 13/514

(54) **UNDERPANTS-TYPE DISPOSABLE DIAPER**

(30) Priority: 11.10.2023 JP 2023176212
(71) Applicant: Unicharm Corporation, Ehime 799-0111 (JP)
(72) Inventor: OHTSUBO, Toshifumi, Kanonji-shi, Kagawa 769-1602 (JP); DANG, Thi Quyen, Ninh Province (VN)
(74) Representative: Dolleymores
(86) International application number: PCT/JP2024/035107
(87) International publication number: WO 2025/079462

(57) **Abstract**

An underpants-type disposable diaper (1) comprising an absorbent body (10), a ventral-side waist part (20), a dorsal-side waist part (30), and waist elastic members (23, 33) that can expand and contract in the lateral direction, wherein: the waist elastic members (23, 33) are provided between a skin-side sheet and a non-skin-side sheet; the dorsal-side waist part (30) has openings (50) that penetrate the skin-side sheet (31) and the non-skin-side sheet (32) in the thickness direction of the sheets; the openings (50) overlap an absorbent core (11) in the lateral direction, and are positioned at the upper side of the absorbent core (11) in the vertical direction; the openings (50) overlap at least one waist elastic member (33) in the vertical direction; and the waist elastic member (33) is discontinuous, at the openings (50), in the lateral direction.

## Description

### FIELD

The present invention relates to an underpants-shaped disposable diaper.

### BACKGROUND

An absorbent article such as a diaper include open holes in the waist portion in order to enhance breathability conventionally has been known. For example, Patent Document 1 discloses an absorbent article in which, in the lengthwise direction, through holes overlap with elastic members and, in the transverse direction, the elastic members are discontinuous within the through holes.

### [CITATION LIST]

### [PATENT LITERATURE]

[Patent Document 1] WO2017/175289

### SUMMARY

### [TECHNICAL PROBLEM]

During wearing of an absorbent article such as diaper or the like, areas such as the wearer's back and the cleft of the buttocks (gluteal cleft) tend to accumulate sweat and moisture and are prone to skin problems. Although, for enhancing the ventilation of the absorbent article, there are absorbent articles having through holes at both sides of the waist portion (both side portions), as in Patent Document 1, but on both sides of the waist portion, there are no through holes along the extension of the gluteal cleft, making it difficult to eliminate accumulation of sweat in the gluteal cleft. Moreover, even in a diaper having multiple small holes on the back or a diaper having through holes on the crotch side of the waist portion, small holes likewise make it difficult to prevent sweat from accumulating on the back and in the gluteal cleft, and the through holes located on the crotch side may increase the risk of leakage. Further, when an open hole is provided on the back side of a diaper, there is a risk of anxiety that leakage occurs from the open hole during wear.

The present invention is achieved in light of conventional problems such as that described above, an aspect of the present invention is to provide an underpants-shaped disposable diaper that improves breathability, makes it less likely for sweat to accumulate on the wearer's back or the gluteal cleft, while also being less likely to leak.

### [SOLUTION TO PROBLEM]

A main aspect of the present invention of achieving the above-described aspect is an underpants-shaped disposable diaper having an up-down direction, a lateral direction, and a thickness direction that are orthogonal to one another, the underpants-shaped disposable diaper including: an absorbent main body that has an absorbent core; a front waist portion; a back waist portion; and a plurality of waist elastic members that are arranged spaced apart in the up-down direction and at least in the back waist portion, the waist elastic members being capable of stretching/contracting in the lateral direction, wherein the back waist portion has a skin-side sheet and a non-skin-side sheet that is located on a non-skin side relative to the skin-side sheet, the waist elastic members are provided between the skin-side sheet and the non-skin-side sheet, the back waist portion has an opening that penetrates the skin-side sheet and the non-skin-side sheet in the thickness direction, the opening is located at a position that overlaps the absorbent core in the lateral direction and is above the absorbent core in the up-down direction, in the up-down direction, the opening overlap at least one of the waist elastic members, and in the lateral direction, the waist elastic member is discontinuous within the opening. Features of the present invention other than the above will become clear by reading the description of the present specification with reference to the accompanying drawings.

### [ADVANTAGEOUS EFFECT OF THE INVENTION]

According to the present invention, it is possible to provide an underpants-shaped disposable diaper that improves breathability, makes it less likely for sweat to accumulate on the wearer's back or the gluteal cleft, while also being less likely to leak.

### BRIEF DESCRIPTION OF DRAWINGS

FIG. 1 is a schematic perspective view of an underpants-shaped disposable diaper 1 as seen from the back.
FIG. 2 is a schematic plan view of the diaper 1 in an unfolded state and stretched state, as viewed from the skin side.
FIG. 3 is a schematic cross-sectional view taken along line A-A in FIG. 2.
FIG. 4A is a diagram showing a portion of a strip-shaped back waist member 300 in the stretched state before forming slits S (openings 50), FIG. 4B is a diagram showing a portion of the strip-shaped back waist member 300 in the stretched state when the slits S (openings 50) has been formed, and FIG. 4C is a diagram showing a portion of a back waist portion 30 in a worn state.
FIG. 5 is a partial enlarged view of the back waist portion 30 in the worn state
FIG. 6A is a partial enlarged view of the back waist portion 30 in FIG. 2 illustrating the openings 50.
FIG. 6B is a diagram illustrating a modified example of the arrangement of a first opening 50a and a second opening 50b.
FIG. 6C is a diagram illustrating waist elastic members 33 that are located in the back waist portion 30 and colored in a specified color.
FIG. 7A is a schematic plan view showing the back waist portion 30 in FIG. 2 and illustrating the back barrier cuff 30RG.
FIG. 7B is a diagram illustrating the back barrier cuff 30RG using a cross section of the diaper 1 taken along line B-B in FIG. 7A.
FIG. 8 is a schematic cross-sectional view of the diaper 1 in an underpants-shaped state when being cut in the lateral center of the diaper 1.
FIG. 9A is a schematic perspective view and schematic cross-sectional view illustrating a modified example in which the back waist portion 30 includes a separated sheet member 70.
FIG. 9B is a diagram illustrating the state of the openings 50 of the diaper 1 of the modified example in the worn state, as viewed from the non-skin side.
FIG. 10 is a diagram illustrating a modified example 1 of a separated sheet member 70 located in the back waist portion 30.
FIG. 11 is a diagram illustrating a modified example 2 of the separated sheet member located in the back waist portion 30.
FIG. 12 is a diagram illustrating a modified example 3 of the separated sheet member 70 located in the back waist portion 30.

### DESCRIPTION OF EMBODIMENTS

At least the following matters are disclosed in the description of this specification and the attached drawings.

Aspect 1 is an underpants-shaped disposable diaper having an up-down direction, a lateral direction, and a thickness direction that are orthogonal to one another, the underpants-shaped disposable diaper including: an absorbent main body that has an absorbent core; a front waist portion; a back waist portion; and a plurality of waist elastic members that are arranged spaced apart in the up-down direction and at least in the back waist portion, the waist elastic members being capable of stretching/contracting in the lateral direction, wherein the back waist portion has a skin-side sheet and a non-skin-side sheet that is located on a non-skin side relative to the skin-side sheet, the waist elastic members are provided between the skin-side sheet and the non-skin-side sheet, the back waist portion has an opening that penetrates the skin-side sheet and the non-skin-side sheet in the thickness direction, the opening is located at a position that overlaps the absorbent core in the lateral direction and is above the absorbent core in the up-down direction, in the up-down direction, the opening overlap at least one of the waist elastic members, and in the lateral direction, the waist elastic member is discontinuous within the opening.

According to the underpants-shaped disposable diaper of Aspect 1, since the opening overlaps the waist elastic member in the up-down direction, the contraction of this elastic member opens up the opening, improving the breathability. The presence of the opening in the area of the back waist portion that overlaps the absorbent core in the lateral direction makes it easier for air to flow to the wearer's back and gluteal cleft, where moisture tends to accumulate, making it less likely for sweat to accumulate. Furthermore, by having the opening located above the absorbent core, excrement becomes less likely to leak.

Aspect 2 is an underpants-shaped disposable diaper having an up-down direction, a lateral direction, and a thickness direction that are orthogonal to one another, the underpants-shaped disposable diaper including: an absorbent main body that has an absorbent core; a front waist portion; a back waist portion; and a plurality of waist elastic members that are arranged spaced apart in the up-down direction and at least in the back waist portion, the waist elastic members being capable of stretching/contracting in the lateral direction, wherein the back waist portion has a skin-side sheet and a non-skin-side sheet that is located on a non-skin side relative to the skin-side sheet, the waist elastic members are provided between the skin-side sheet and the non-skin-side sheet, the back waist portion has an opening that penetrates the skin-side sheet and the non-skin-side sheet in the thickness direction, in the up-down direction, the opening overlap at least one of the waist elastic members, in the lateral direction, the waist elastic member is discontinuous within the opening, the back waist portion includes a back barrier cuff in an upper end portion, in the up-down direction, of the absorbent main body, the back barrier cuff has a base end portion being incapable of standing up, and a standing portion being capable of standing up on a skin side in the thickness direction due to a barrier-cuff elastic member that contracts in the lateral direction using the base end portion as a fulcrum, the standing portion is located below the base end portion in the up-down direction, and the base end portion is located below the opening in the up-down direction.

According to the underpants-shaped disposable diaper of Aspect 2, in the back barrier cuff, the standing portion, which is a free part, is located below (on the crotch side of) the base end portion, that is, the standing portion extends from the base end portion toward the crotch side and stands up when worn, and therefore urine, feces, etc. excreted in the crotch portion is prevented from spreading to the back waist portion. Therefore, even with the opening located in the back waist portion, anxiety that leakage occurs can be reduced by having such a back barrier cuff located below the opening.

Aspect 3 is the underpants-shaped disposable diaper according to Aspect 1 or 2, wherein a length, in the up-down direction, of the opening is less than 15 mm.

According to the underpants-shaped disposable diaper of Aspect 3, since the length, in the up-down direction, of the opening is less than 15 mm, the user's finger is difficult to enter into the opening when putting on the diaper, thereby reducing the risk of the diaper tearing and the fit decreasing due to the finger entering into the opening.

Aspect 4 is the underpants-shaped disposable diaper according to any one of Aspects 1 to 3, wherein in the stretched state, the opening is a slit extending along the up-down direction.

According to the underpants-shaped disposable diaper of Aspect 4, the fact that the opening is a slit extending in the lateral direction makes the end portions of the slit more likely to tear when pulling up the diaper at the time of putting on, causing a risk that the fit deteriorates, making leakage more likely to occur. In this regard, such a risk can be prevented by the opening being the slit extending in the up-down direction, which is the direction in which the diaper is pulled up.

Aspect 5 is the underpants-shaped disposable diaper according to any one of Aspects 1 to 3, wherein in the stretched state, the opening is a cutout formed by cutting out a specified range.

According to the underpants-shaped disposable diaper of Aspect 5, the opening becomes easier to open, improving breathability. As a result, the diaper becomes more resistant to dampness.

Aspect 6 is the underpants-shaped disposable diaper according to any one of Aspects 1 to 5, wherein when one of the waist elastic members that overlap the opening in the up-down direction is defined as an overlapping waist elastic member, in the stretched state, a length, in the up-down direction, of the opening is longer than a distance in the up-down direction between the overlapping waist elastic member and another waist elastic member that is adjacent to the overlapping waist elastic member in the up-down direction.

According to the underpants-shaped disposable diaper of Aspect 6, Since the length, in the up-down direction, of the opening is longer than the distance between the waist elastic members adjacent in the up-down direction, at least one waist elastic member is discontinuous within the opening. Due to the contraction of the overlapping waist elastic member, the opening opens up in the lateral direction, making the diaper less likely to become damp.

Aspect 7 is the underpants-shaped disposable diaper according to Aspect 6, wherein when the waist elastic member that is located above and adjacent to the overlapping waist elastic member in the up-down direction is defined as an upper waist elastic member, and the waist elastic member that is located below the overlapping waist elastic member is defined as a lower waist elastic member, in the stretched state, the length, in the up-down direction, of the opening is longer than a distance in the up-down direction from the upper waist elastic member to the lower waist elastic member.

According to the underpants-shaped disposable diaper of Aspect 7, since the length of the opening is longer than the distance between the adjacent elastic members (upper waist elastic member and lower waist elastic member) that are adjacent in the up-down direction to the overlapping waist elastic member overlapping the opening in the up-down direction (i.e., two intervals between adjacent elastic members), at least two waist elastic members are discontinuous within the opening. As a result, the opening opens wider, improving breathability and making the diaper further less likely to become damp.

Aspect 8 is the underpants-shaped disposable diaper according to any one of Aspects 1 to 6, wherein a magnitude of force required to stretch the waist elastic member that overlaps the opening in the up-down direction, by a unit length in the lateral direction is greater than a magnitude of force required to stretch the waist elastic member that does not overlap the opening in the up-down direction, by the unit length in the lateral direction.

According to the underpants-shaped disposable diaper of Aspect 8, because the waist elastic member that overlaps the opening in the up-down direction has a strong tightening force, even in the presence of the opening, the lateral constriction is less likely to weaken, and the fit can be maintained.

Aspect 9 is the underpants-shaped disposable diaper according to any one of Aspects 1 to 8, wherein in the up-down direction, the opening overlaps at least two of the waist elastic members, the at least two waist elastic members being adjacent in the up-down direction, and in the stretched state, a distance in the up-down direction between the two waist elastic members adjacent in the up-down direction is shorter than a distance the up-down direction between another two of the waist elastic members, the other two waist elastic members being adjacent in the up-down direction and not overlapping the opening in the up-down direction.

According to the underpants-shaped disposable diaper of Aspect 9, the more waist elastic members overlapping the opening in the up-down direction, the weaker the lateral tightening force in the area where the opening is provided tends to become. However, by reducing the distance between the elastic members overlapping the opening in the up-down direction, the tightening force can be increased and the fit can be maintained. Further, as the number of the elastic members overlapping the opening in the up-down direction increases, the opening is pulled in the lateral direction, making it easier for it to open.

Aspect 10 is the underpants-shaped disposable diaper according to Aspect 9, wherein the plurality of openings that overlap the two waist elastic members adjacent in the up-down direction are provided space apart in the lateral direction.

According to the underpants-shaped disposable diaper of Aspect 10, in the area that overlaps the above-mentioned openings in the up-down direction and where the distance between the elastic members is short, the tightening force tends to increase, but by having the plurality of openings, the pulling force of the elastic members can be reduced, making it possible to achieve a better fit without overtightening.

Aspect 11 is the underpants-shaped disposable diaper according to any one of Aspects 1 to 10, wherein in the up-down direction, the opening is provided in a lower region of an area obtained by bisecting between an upper end of the absorbent main body and an upper end of the back waist portion.

According to the underpants-shaped disposable diaper of Aspect 11, the lower region mentioned above is located close to the wearer's gluteal cleft, and therefore, the presence of the opening in such a region facilitates ventilation, thereby reducing the accumulation of sweat in the gluteal cleft.

Aspect 12 is the underpants-shaped disposable diaper according to any one of Aspects 1 to 11, wherein at least two of the openings are provided spaced apart in the lateral direction, when one of the two openings located on a one side in the lateral direction is defined as a first opening, and the other of the two openings located on another side in the lateral direction is defined as a second opening, in the stretched state, a distance in the lateral direction between an upper end of the first opening and an upper end of the second opening is shorter than a length, in the up-down direction, of the opening, and the first opening and the second opening have an overlapping portion in the up-down direction, or the first opening and the second opening are spaced apart in the up-down direction, and a distance from a lower end of the upper opening of the first and second openings to an upper end of the lower opening of the first and second openings is shorter than the length, in the up-down direction, of the opening.

According to the underpants-shaped disposable diaper of Aspect 12, even if each one opening is small, at least two openings are located closely, making it easier to visually recognize them. In addition, since each opening is small, it is difficult for the use's fingers to enter inside, reducing the risk of the opening tearing.

Aspect 13 is the underpants-shaped disposable diaper according to Aspect 12, wherein the back waist portion has an opening group including the first opening and the second opening, a plurality of the opening groups are provided spaced apart in the lateral direction, and in the stretched state, a distance in the lateral direction between two of the opening groups that are adjacent in the lateral direction is longer than the length, in the up-down direction, of the opening.

According to the underpants-shaped disposable diaper of Aspect 13, by providing the plurality of opening groups, the visibility of the openings can be improved and further the breathability can be enhanced. If the distance between the opening groups is excessively narrow, many discontinuous parts occur in the waist elastic members, which may weaken the tightening force. Therefore, by not making the opening groups excessively close, deterioration of the tightening force can be avoided.

Aspect 14 is the underpants-shaped disposable diaper according to Aspect 12 or 13, wherein in the stretched state, the first opening and the second opening are separated in the up-down direction by equal to or more than half the length, in the up-down direction, of the opening.

According to the underpants-shaped disposable diaper of Aspect 14, the distance in the lateral direction between the first opening and the second opening is narrow, and therefore if the distance in the up-down direction between the two openings is also narrow, there is a risk that the openings are less likely to open properly. However, by spacing apart the openings 50 in the up-down direction, the difficulty in opening can be avoided.

Aspect 15 is the underpants-shaped disposable diaper according to any one of Aspects 1 to 14, wherein a plurality of the openings are provided, and the plurality of openings do not overlap each other in the lateral direction.

According to the underpants-shaped disposable diaper of Aspect 15, the plurality of openings overlap each other in the lateral direction, when pulling up the diaper at the time of putting on, there is a risk that tears occur between the openings, causing the fit to deteriorate. However, since the openings do not overlap each other in the lateral direction, tearing is less likely to occur.

Aspect 16 is the underpants-shaped disposable diaper according to any one of aspects 1 to 15, wherein a post-treatment tape that is used to discard the underpants-shaped disposable diaper after use is provided on a non-skin-side face of the back waist portion, and the opening does not overlap the post-treatment tape in the lateral direction.

According to the underpants-shaped disposable diaper of Aspect 16, by ensuring that the opening does not overlap the post-treatment tape, it becomes easier to fasten the post-treatment tape.

Aspect 17 is the underpants-shaped disposable diaper according to Aspect 2, wherein the opening is provided outside the absorbent core on both sides in the lateral direction.

According to the underpants-shaped disposable diaper of Aspect 17, since the opening is provided not only in the area that overlap the absorbent core in the lateral direction, but also in the areas outside the absorbent core on both sides in the lateral direction, the stiffness is reduced in the area where the opening exist in the lateral direction, making it more likely to deform This separates the movement of the upper part of the back waist portion from the movement of the back barrier cuff, making it easier for the back barrier cuff to follow the movement of the buttocks and less likely to slip.

Aspect 18 is the underpants-shaped disposable diaper according to Aspect 2 or 17, wherein a lower end, in the up-down direction, of the base end portion of the back barrier cuff is located below an upper end, in the up-down direction, of the absorbent main body.

According to the underpants-shaped disposable diaper of Aspect 18, since the absorbent main body exists below (on the crotch side of ) the lower end of the base end portion, the discharged liquid blocked by the back barrier cuff is more likely to be absorbed, making it less likely to leak.

Aspect 19 is the underpants-shaped disposable diaper according to any one of Aspects 2, 17, and 18, wherein the front waist portion and the back waist portion are joined by a pair of side joining portions in both side portions in the lateral direction, both side portions, in the lateral direction, of the back barrier cuff are joined by the pair of side joining portions, and in the back barrier cuff, the pair of side joining portions are not fixed between a one-side end and an other-side end in the lateral direction.

According to the underpants-shaped disposable diaper of Aspect 19, since, in the back barrier cuff, he area between the pair of side joining portions is not fixed, the back barrier cuff can move freely between the pair of side joining portions, making it easier for the standing portion to stand up. By the standing portion standing up so as to be in sufficiently contact with the skin, a pocket-like space is created, making it less likely to leak.

Aspect 20 is the underpants-shaped disposable diaper according to any one of Aspects 2 and 17 to 19, wherein a magnitude of force required to stretch the barrier-cuff elastic member by a unit length in the lateral direction is smaller than a magnitude of force required to stretch the waist elastic member that overlaps the opening in the up-down direction, by the unit length in the lateral direction.

According to the underpants-shaped disposable diaper of Aspect 20, since the force required to stretch the barrier-cuff elastic member that is placed in the back barrier cuff is small, the back barrier cuff is more likely to deform, thereby making the diaper easier to fit to the wearer's buttocks.

Aspect 21 is the underpants-shaped disposable diaper according to any one of Aspects 1 to 20, wherein the opening does not penetrate the back waist portion in the thickness direction.

According to the underpants-shaped disposable diaper of Aspect 21, although there are the plurality of openings, the fact that the openings do not penetrate the back waist portion can reduce anxiety of leakage.

Aspect 22 is the underpants-shaped disposable diaper according to any one of Aspects 1 to 21, wherein the underpants-shaped disposable diaper includes a separated sheet member that is separate from the skin-side sheet and the non-skin-side sheet, and the separated sheet member covers the opening from a skin side.

According to the underpants-shaped disposable diaper of Aspect 22, the separated sheet member covers the opening from the skin side, making it possible to reduce the risk of leakage from the opening.

Aspect 23 is the underpants-shaped disposable diaper according to Aspect 22, wherein the separated sheet member is a sweat-absorbing sheet.

According to the underpants-shaped disposable diaper of Aspect 23, sweat absorbed by the sweat-absorbing sheet evaporates through the opening, which is located on the non-skin side relative to the sweat-absorbing sheet, making the diaper less likely to damp.

Aspect 24 is the underpants-shaped disposable diaper according to Aspect 22, wherein the separated sheet member is nonwoven fabric having an opening that is smaller than the opening.

According to the underpants-shaped disposable diaper of Aspect 24, in the parts of the opening, the diaper becomes less likely to damp because the opening of the nonwoven fabric is not blocked. Furthermore, when the underpants-shaped disposable diaper is viewed from the non-skin side, not only the nonwoven fabric but also the smaller opening of the nonwoven fabric can be seen through the opening, enabling to confirm having good breathability and to give cool impression.

Aspect 25 is the underpants-shaped disposable diaper according to any one of Aspects 1 to 24, wherein a lower end, in the up-down direction, of the opening is located below an upper end, in the up-down direction, of the absorbent main body in the front waist portion.

According to the underpants-shaped disposable diaper of aspect 25, on the back waist portion side, the dampness in the wearer's back and gluteal cleft is prevented by placing the opening near the gluteal cleft, whereas, on the front waist portion side, urine leakage is prevented by placing the absorbent main body up to the upper part.

### Embodiment

Hereinafter, an embodiment of an underpants-shaped disposable diaper according to the present invention will be described below, using an underpants-shaped disposable diaper 1 for infants (hereinafter also referred to as a "diaper 1") as an example. However, the underpants-shaped absorbent article according to the present invention is not limited to a disposable diaper for infants, and it may be a disposable diaper for adults.

### Basic Configuration of Underpants-shaped disposable diaper 1

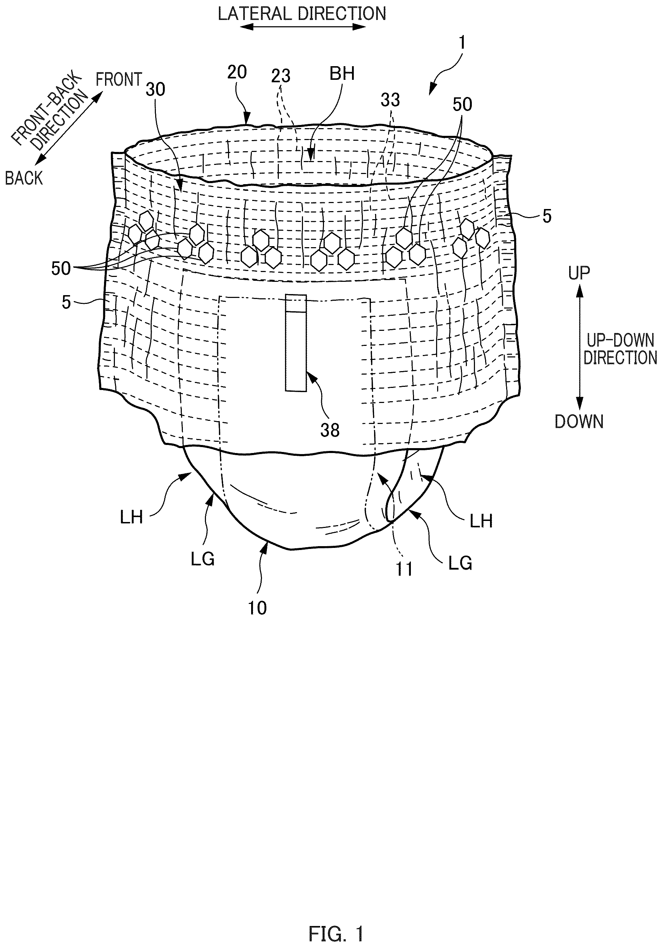

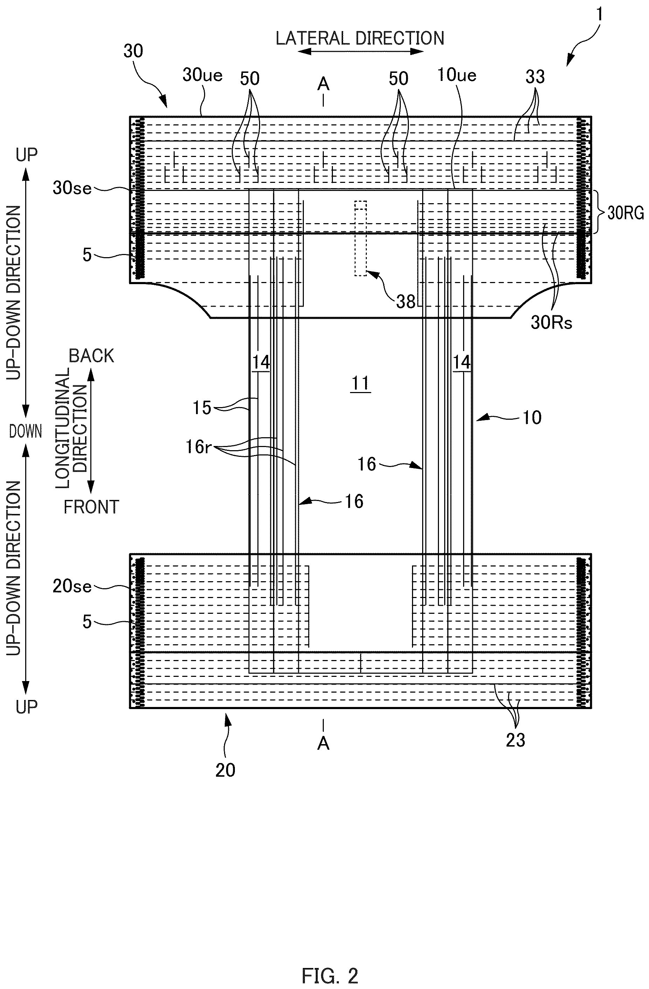

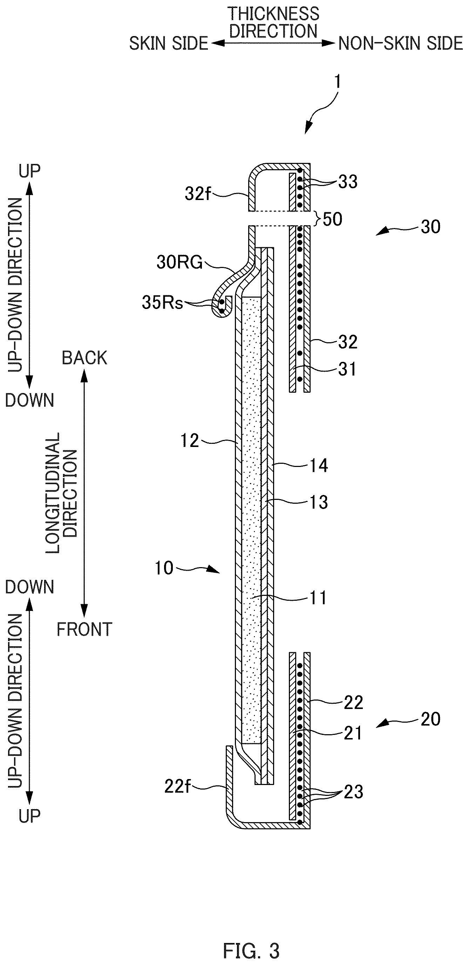

FIG. 1 is a schematic perspective view of the underpants-shaped disposable diaper 1 as seen from the back. FIG. 2 is a schematic plan view of the diaper 1 in an unfolded state and stretched state, as viewed from the skin side. FIG. 3 is a schematic cross-sectional view taken along line A-A in FIG. 2.

The diaper 1 in an underpants-shaped state (worn state) shown in FIG. 1 has an up-down direction, a lateral direction, and a front-back direction that intersect with one another, and the diaper 1 has a waist opening BH and a pair of leg openings LH. In an up-down direction, the waist opening BH side is referred to as the upper side, and the crotch side is referred to as the lower side. Also, as shown in FIG. 3, the direction in which the constituent materials of the diaper 1 are stacked is referred to the thickness direction, and, in the thickness direction, the side that comes into contact with the wearer is referred to as the skin side, and the side that does not come into contact with the wearer is referred to as the non-skin side.

The unfolded state of the diaper 1 refers to a state in which the pair of joining portions 5 provided in the both side portions of the diaper 1 are separated from each other, and the diaper 1 is opened and laid out flat. Further, the stretched state of the diaper 1 shown in FIG. 2 refers to a state in which the elastic members provided in the diaper 1 has stretched to the extent that wrinkles in the diaper 1 are no longer visible. More specifically, it refers to the state in which the dimensions of the constituent members of the diaper 1 are stretched until they match or are close to the dimensions of the members alone (i.e., the dimensions in the state where the stretchability of the elastic members is not exhibited), and also means the state in which the elastic members between later-described openings 50 adjacent in the lateral direction have stretched until the stretchability is not exhibited.

The diaper 1 in the present embodiment is a so-called three-piece pants-type diapers, and includes an absorbent main body 10, a front waist portion 20, and a back waist portion 30. The front waist portion 20 is a portion that abuts the wearer's ventral portion, and the back waist portion 30 is a portion that abuts the wearer's dorsal portion.

In the diaper 1 in the unfolded state shown in FIG. 2, the front waist portion 20 and the back waist portion 30 are arranged so that their longitudinal direction is aligned with the lateral direction of the diaper 1. The longitudinal one-side end portion of the absorbent main body 10 is located in the central portion, in the lateral direction, of the front waist portion 20, and the longitudinal other-side end portion of the absorbent main body 10 is located in the central portion, in the lateral direction, of the back waist portion 30.

The absorbent main body 10 is folded in two at approximately the longitudinal center of the absorbent main body 10 such that the longitudinal direction of the absorbent main body 10 is aligned with the up-down direction of diaper 1. Then, the both side end portions 20se, 20se of the front waist portion 20 and the both side end portions 30se, 30se of the back waist portion 30 , in the lateral direction of diaper 1 are joined by thermal welding, ultrasonic welding, or the like, thereby forming the pair of side joining portions 5, obtaining the diaper 1 in the underpants-shaped state as shown in FIG. 1.

As shown in FIG. 3, the absorbent main body 10 includes: an absorbent core 11 that absorbs excrement; a liquid-permeable top sheet that is located on the skin side relative to the absorbent core 11; a liquid-impermeable back sheet 13 that is located on the non-skin side relative to the absorbent core 11; and exterior sheets 14 that are located on the non-skin side relative to the back sheet 13, and these members are bonded together using an adhesive or the like.

As the absorbent core 11, a product made of liquid-absorbent fibers such as pulp fibers containing superabsorbent polymer (SAP) and molded into a specified shape can be exemplified. The absorbent core 11 may be covered with a core-wrapping sheet (not shown) such as liquid-permeable tissue paper or nonwoven fabric. The top sheet 12 is formed, for example, by nonwoven fabric, such as air-through nonwoven fabric and spunbond nonwoven fabric. The back sheet 13 is a film having a liquid impermeability and breathability, and, for example, made of polyethylene, polypropylene, or the like. The exterior sheet 14 is formed, for example, by nonwoven fabric which is equivalent to the top sheet 12.

In addition, the absorbent main body 10 has leg elastic members 15 (e.g., elastic strings) that stretch/contract in the longitudinal direction (up-down direction), on the both side portions in the lateral direction. This allows the diaper 1 to fit around the wearer's legs.

Also, the absorbent main body 10 has leak-proof wall portions 16 that can stand on the skin side, in the both side portions in the lateral direction. For example, the leak-proof wall portions 16 are formed as follows: the both side portions, in the lateral direction, of the exterior sheet 14 are folded back inward in the lateral direction so as to be located on the skin-side face of the top sheet 12, and leak-proof-wall elastic members 16r (e.g., elastic strings) that stretch/contract in the longitudinal direction are provided in the folded-back portions.

As shown in FIG. 3, the front waist portion 20 and the back waist portion 30 each have a skin-side sheet 21 and 31 and a non-skin-side sheet 22 and 32, which are made of flexible nonwoven fabric, and the like. Also, as shown in FIG. 3, in order to improve the texture and durability, in the upper part of the front waist portion 20, the non-skin-side sheet 22 is folded back from the upper end of the front waist portion 20 downward to the skin side, thereby forming the fold-back portion 22f. Similarly, in the upper part of the back waist portion 30, the non-skin-side sheet 32 is folded back from the upper end of the back waist portion 30 downward to the skin side, thereby forming the fold-back portion 32f. In addition, the back waist portion 30 has a back barrier cuff 30RG, which is formed by the lower part of the fold-back portion 32f. Details of the back barrier cuff 30RG will be described later.

Further, the back waist portion 30 has a plurality of openings 50s that have a specified length in the up-down direction and penetrate the skin-side sheet 31 and non-skin-side sheet 32 that form the back waist portion 30 from the skin side to the non-skin side in the thickness direction. Details of the openings 50 will be described later.

Between the skin-side sheet 21 (31) and the non-skin-side sheet 22 (32) of the front waist portion 20 (back waist portion 30) serving as the waist portion, a plurality of waist elastic members 23 (33) (e.g., elastic strings) that stretch/contract in the lateral direction are located. The waist elastic members 23 (33) are fixed to the skin-side sheet 21 (31) and the non-skin-side sheet 22 (32) by adhesive or ultrasonic bonding, in a state of stretching in the lateral direction. More specifically, the plurality of waist elastic members 23 (33) that are coated with adhesive are arranged between the skin-side sheet 21 (31) and the non-skin-side sheet 22 (32), in a state of stretching in the lateral direction, and thereby the waist elastic members 23 (33) are joined and fixed in a state of stretching to the skin-side sheet 21 (31) and the non-skin-side sheet 22 (32). The plurality of waist elastic members 23, 33 are arranged spaced apart in the up-down direction in the front waist portion 20 and the back waist portion 30. Thus, the front waist portion 20 and the back waist portion 30 can stretch/contract in the lateral direction, to fit around the wearer's waist.

In addition, some of the waist elastic members 23 (33) are made discontinuous in a region that overlaps the absorbent core 11 located near the center in the lateral direction, thereby to prevent stretchability from exhibiting. This suppresses the contraction in the lateral direction acting on the absorbent core 11, making it easier for the absorbent core 11 to be maintained approximately flat, thereby suppressing leakage of excrement, etc.

Although the basic configuration of the diaper 1 is described above, the configuration of the diaper 1 is not limited to the above. For example, the diaper 1 may be a two-piece diaper, and the front waist portion 20 and the back waist portion 30 may be formed of a single continuous member, or an exterior member may be provided to connect the front waist portion 20 and the back waist portion 30. Also, the leg elastic members 15 may be an elastic member in sheet form (stretchable film or stretchable nonwoven fabric) instead of elastic strings. Further, in the back waist portion 30, a sheet-like elastic member (stretchable film or stretchable nonwoven fabric) may be provided above the positions where the openings 50 are provided.

### Openings

First, the following describes the formation of the openings 50 in the present embodiment. The openings 50, which are provided in the back waist portion 30, are formed during the manufacturing process of the back waist portion 30 in the manufacture of the diaper 1. The back waist portion 30 is formed by dividing a strip-shaped back waist member 300 into individual back waist portions 30, the strip-shaped back waist member 300 including: starting from the skin side, a strip-shaped non-skin-side-sheet fold-back portion 320f in which a strip-shaped non-skin-side sheet 320 is folded back; a strip-shaped skin-side sheet (not shown); continuous waist elastic members 330; and a strip-shaped non-skin-side sheet (not shown).

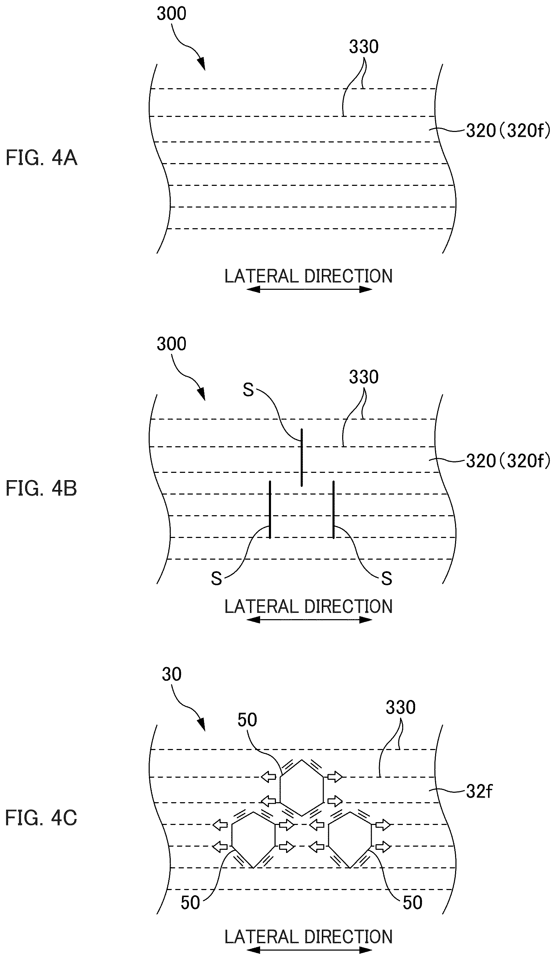

FIG. 4A is a diagram showing a portion of the strip-shaped back waist member 300 in the stretched state before forming slits S (openings 50). FIG. 4B is a diagram showing a portion of the strip-shaped back waist member 300 in the stretched state when the slits S (openings 50) has been formed. FIG. 4C is a diagram showing a portion of the back waist portion 30 in the worn state. FIGS. 4A, 4B, and 4C are diagrams as viewed from the skin side.

FIG. 4A shows the strip-shaped back waist member 300 before slits S are formed, in the state where the strip-shaped back waist member 300 has stretched until wrinkles in the strip-shaped non-skin-side-sheet fold-back portion 320f, the strip-shaped skin-side sheet, and the strip-shaped non-skin-side sheet are substantially eliminated.

Next, as shown in FIG. 4B, linear slits S extending along the up-down direction are formed in the strip-shaped back waist member 300 that is in the stretched state so as to cross some of the continuous waist elastic members 330. Each slit S is formed by cutting two continuous waist elastic members 330 together with the strip-shaped skin-side sheet and the strip-shaped non-skin-side sheet, using a specified blade. FIG. 4B shows the moment when the slits S are formed by cutting. The slits S becomes into the openings 50 as shown in FIG. 4C, due to the continuous waist elastic members 330.

FIG. 4C shows a part of the back waist portion 30 when the wearer is wearing the diaper 1. The openings 50 of the back waist portion 30 in the worn state each have an outline in which on both sides in the lateral direction open, making the shape of each opening 50 approximately hexagonal. This is because, as shown in FIG. 4C, the waist elastic members 33 contract from the stretched state, pulling the perimeter of the opening 50 in the lateral direction. As a result, in the worn state, the opening 50 spreads out larger, making it possible to improve the breathability of the diaper 1. By making each one opening 50 open more widely, it is possible to secure the breathability of the diaper 1 with fewer openings 50. Furthermore, even before wearing, the waist elastic members 33 in a contracted state can make the openings 50 open in the lateral direction, and, as a result, parents who put the diaper 1 on a wearer (e.g., an infant) can visually recognize the openings 50 and recognize the improvement in breathability.

As mentioned above, the shape of the openings 50 in the present embodiment is approximately hexagonal, but strictly speaking it is not a hexagon, but a shape in which each vertex can be recognized, and most parts of its contours are curved rather than straight. Furthermore, in cases where the number of the continuous waist elastic members 330 that are cut in the process of forming slits S is different, the number of the elastic members pulling the periphery of the openings 50 to the left and right when forming the openings is also different, and the shape of the openings will also change.

The formation of the slits S may be carried out after the absorbent main body 10 is placed in the back waist portion 30, or the absorbent main body 10 may be placed after the slits S are formed in the back waist portion 30.

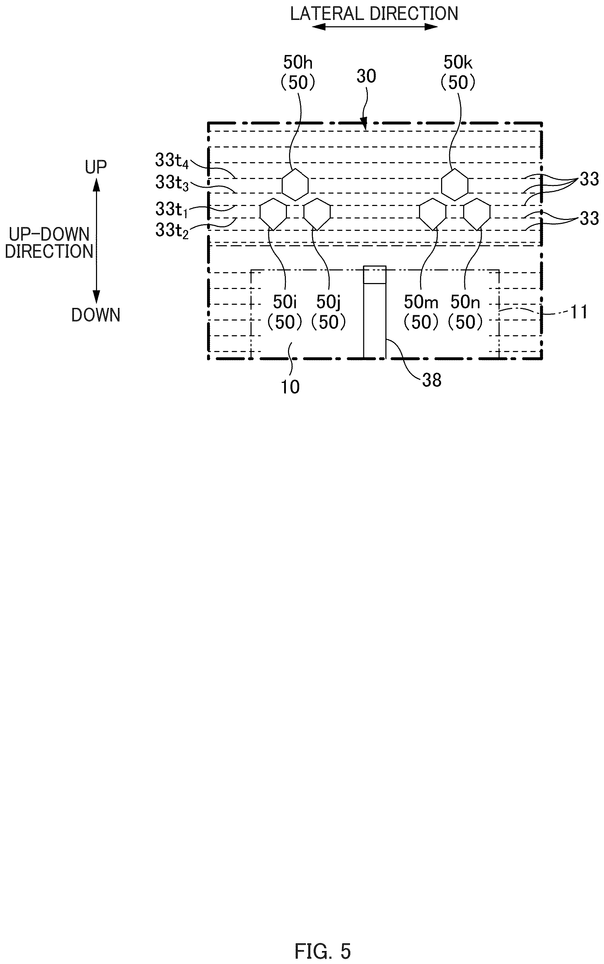

Next, the configuration of the openings 50 will be explained. FIG. 5 is a partial enlarged view of the back waist portion 30 in the worn state. As shown in FIGS. 2 and 5, the plurality of openings 50 of the back waist portion 30 are provided in a part of the back waist portion 30 that is located above the absorbent core 11 in the up-down direction. More specifically, the plurality of openings 50 of the back waist portion 30 are provided so as to be located in a part of the back waist portion 30 that is located above the absorbent main body 10 in the up-down direction, and no opening 50 is provided in an area overlapping with the absorbent main body 10 in a planar view. In the lateral direction, the openings 50 are provided at positions that overlap the absorbent core 11 and positions located outside the absorbent core 11. It is sufficient that the openings 50 are provided at least so as to overlap the absorbent core 11.

Furthermore, the openings 50 in the present embodiment each overlap at least two waist elastic members 33 in the up-down direction, and these waist elastic members 33 are discontinuous in the lateral direction within the opening 50. Specifically, the waist elastic members 33 that have been cut are not provided inside each opening 50, and the cut waist elastic members 33 are discontinuous in the lateral direction within the opening 50. This is because, when forming the opening 50, the waist elastic members 33 provided at the formation position of the opening 50 are cut off together. Therefore, as shown in FIG. 5, for example, the waist elastic member 33t₁ is continuous from outside the opening 50i in the lateral direction to outside the opening 50j in the lateral direction, and is not located inside the opening 50i and the opening 50j. Furthermore, the waist elastic member 33t₁ continues from outside the opening 50j in the lateral direction to outside the opening 50m in the lateral direction, and also continues from outside the opening 50m in the lateral direction to outside the opening 50n in the lateral direction, and is not located inside the opening 50m and the opening 50n. The waist elastic member 33t₂ has the same configuration as the waist elastic member 33t₁. Similarly, the waist elastic member 33t₃ and 33t₄ are continuous from outside the opening 50h in the lateral direction to outside the opening 50k in the lateral direction, and are not located inside the opening 50h and the opening 50k.

In this way, since the openings 50 each overlaps the corresponding waist elastic members 33 in the up-down direction, the contraction of the waist elastic members 33 open up each opening 50, making it possible to improve breathability. When wearing the diaper 1, sweat and moisture tend to accumulate on the wearer's back and gluteal cleft, but in the present embodiment, the opening 50 is located in the area of the back waist portion 30 that overlaps the absorbent core 11 in the lateral direction, making it easier for air to flow to the wearer's back and gluteal cleft, where moisture tends to accumulate, making it less likely for sweat to accumulate. Further, in the back waist portion 30, in cases where the openings 50 are located in the lower side (crotch side) in the up-down direction, there is a risk that leakage may occur more easily. However, as in the present embodiment, by providing the openings 50 above the absorbent core 11, the risk of excrement leaking from the openings 50 is reduced.

In addition, in the present embodiment, the openings 50 each overlap at least two waist elastic members 33 in the up-down direction, but the present invention is not limited to this. It is sufficient that the openings 50 each overlap at least one waist elastic member 33, or the opening 50 may overlap three or more waist elastic members 33.

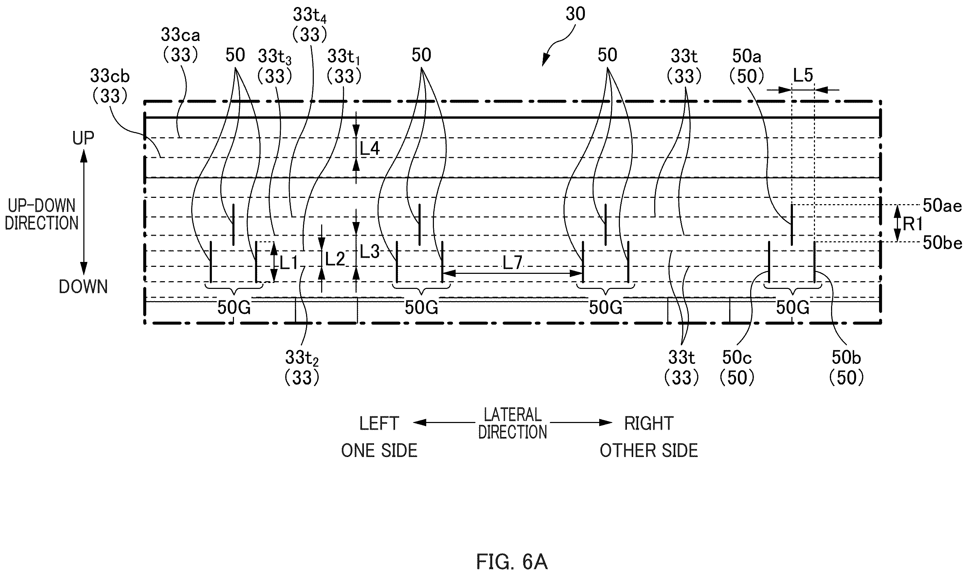

FIG. 6A is a partial enlarged view of the back waist portion 30 in FIG. 2 illustrating the openings 50. As shown in FIG. 6A, in the stretched state, the openings 50 are each a slit extending along the up-down direction. The slit is preferably formed at an angle of about 90 degrees relative to the waist elastic members 33, but may be formed at an angle in the range of about 70 to 110 degrees relative to the waist elastic members 33. In cases where the openings 50 are slits extending in the lateral direction, the end portions of each slit are more likely to tear when pulling up the diaper 1 at the time of putting on, causing a risk that the fit deteriorates, making leakage more likely to occur. In this regard, such a risk can be prevented by the openings 50 being the slits extending in the up-down direction, which is the direction in which the diaper 1 is pulled up.

Furthermore, it is preferable that the length L1, in the up-down direction, of the opening 50 is less than 15 mm. More preferably, the length L1, in the up-down direction, of the opening 50 is greater than 5 mm and less than 15 mm. If the length L1, in the up-down direction, of the opening 50 is less than 15 mm, in a state where the openings 50 are spread out as shown in FIG. 5, fingers become difficult to enter into the openings 50 when putting on the diaper 1, thereby reducing the risk of the diaper 1 tearing and the fit decreasing due to fingers entering into the openings 50. Also, if the openings 50 are excessively small, breathability decreases, and therefore by making the length L1 in the up-down direction greater than 5 mm, it becomes easier to achieve an improvement in breathability.

In the present embodiment, the openings 50 are each a slit extending in the up-down direction, but the present invention is not limited to this. The opening 50 may also be a cutout formed by cutting out a specified area in the stretched state. By forming the opening 50 by cutting out, the opening 50 becomes easier to open, making it possible to improve breathability. This makes the diaper 1 more resistant to dampness. Furthermore, the aesthetic appeal of the diaper 1 can be improved by forming the cutout into a desired shape such as a circle, a star, a heart, or the like.

Next, the relationship between the waist elastic members 33 and the openings 50 is further described below. As shown in FIG. 6A, when one of the waist elastic members 33 that overlap any of the openings 50 in the up-down direction is defined as a waist elastic member (overlapping waist elastic member) 33t₁, in the stretched state, the length L1, in the up-down direction, of the opening 50 is longer than the distance L2 in the up-down direction between the waist elastic member 33t₁ and another waist elastic member 33t₂ adjacent thereto in the up-down direction (L1> L2). Since the length L1, in the up-down direction, of the opening 50 is longer than the distance between the waist elastic members (33t₁, 33t₂) adjacent in the up-down direction, at least one or more of the waist elastic members 33 may be discontinuous within the opening 50. As a result, when putting on the diaper 1, the opening 50 opens in the lateral direction due to the waist elastic member (overlapping waist elastic member) 33t₁, making the diaper 1 less likely to become damp. Here, as the waist elastic members 33t adjacent to each other in the up-down direction, the waist elastic member 33t₁ overlapping the opening 50 and the waist elastic member 33t₂ located therebelow are selected, but for example, the interval between the waist elastic member 33t₁ and a waist elastic member 33t located thereabove may also be used. Further, the case where the waist elastic members 33 are not arranged at equal intervals is also included. Even in such a case, it is desirable that the waist elastic members 33 be positioned such that the length L1, in the up-down direction, of the opening 50 is longer than the distance L2 in the up-down direction between the overlapping waist elastic member 33t₁ and another waist elastic member 33 adjacent thereto in the up-down direction.

Furthermore, when the waist elastic member 33 located above and adjacent to the above-mentioned overlapping waist elastic member 33t₁ in the up-down direction is defined as an upper waist elastic member 33t₃, and the waist elastic member 33 located below is defined as a lower waist elastic member 33t₂, it is preferable that, in the stretched state, the length L1, in the up-down direction, of the opening 50 is longer than the distance L3 in the up-down direction from the upper waist elastic member 33t₃ to the lower waist elastic member 33t₂ (L1 > L3). The length L3 between the waist elastic members 33 adjacent above and below to the overlapping waist elastic member 33t₁ in the up-down direction (upper waist elastic member 33t₃ and lower waist elastic member 33t₂) means two intervals between the waist elastic members 33 adjacent in the up-down direction, and since the length L1, in the up-down direction, of the opening 50 is longer than the distance L3, at least two waist elastic members 33 are discontinuous within the opening 50. As a result, the opening 50 opens wider, improving breathability and making the diaper 1 less likely to become damp.

Further, in the present embodiment, the magnitude of the force required to stretch the waist elastic member 33t, which overlaps the opening 50 in the up-down direction, by unit length in the lateral direction is greater than the magnitude of the force required to stretch the waist elastic member 33, which does not overlap the opening 50 in the up-down direction, by unit length in the lateral direction. In other words, the waist elastic member 33t, which is located at a position overlapping the opening 50 in the up-down direction, has a stronger tightening force in the lateral direction. In this way, because the tightening force of the waist elastic member 33t, which overlaps the opening 50, is strong, even if the waist elastic member 33t has a discontinuous part because of the opening 50, the lateral constriction is less likely to weaken, and the fit of the back waist portion 30 can be maintained.

As described above, in the present embodiment, in the up-down direction, the opening 50 overlaps at least two waist elastic members 33t (e.g., waist elastic members 33t₁ and 33t₂) that are adjacent in the up-down direction, and in the stretched state shown in FIG. 6A, the distance L2 in the up-down direction between these two waist elastic members 33t is shorter than the distance L4 in the up-down direction between two waist elastic members 33ca and 33cb that are adjacent in the up-down direction and do not overlap the opening 50 in the up-down direction (L2 < L4). The more waist elastic members 33 overlapping the opening 50 in the up-down direction, the weaker the lateral tightening force in the area where the opening 50 is located tends to become. However, by reducing the distance L2 between the waist elastic members 33t, 33t overlapping the opening 50 in the up-down direction, the tightening force can be increased.

As a result, the fit of the back waist portion 30 can be maintained. Further, as the number of the elastic members 33t overlapping the opening 50 in the up-down direction increases, the opening 50 is pulled in the lateral direction, making it easier for it to open.

Furthermore, in the present embodiment, as shown in FIG. 6A, the plurality of openings 50 that overlap two waist elastic members 33t₁ and 33t₂ adjacent in the up-down direction are provided spaced apart in the lateral direction. As described above, in the area where the distance L2 between the two waist elastic members 33t₁ and 33t₂ is short, the lateral tightening force tends to increase, but by having the plurality of openings 50 spaced apart in the lateral direction, the pulling force of the waist elastic members 33t₁ and 33t₂ can be reduced, making it possible to achieve a better fit without overtightening. In other words, by cutting the waist elastic members 33t₁, 33t₂, the pulling force of the waist elastic members 33t₁, 33t₂ can be adjusted, thereby ensuring constriction around the waist while reducing the risk of excessive constriction, and improving the fit of the diaper 1.

Preferably, in the up-down direction, the openings 50 are located in the lower region of the area obtained by bisecting between the upper end 10ue of the absorbent main body 10 and the upper end 30ue of the back waist portion 30. The lower area when bisecting is located close to the wearer's gluteal cleft, and therefore, by placing the openings 50 in such a region, it becomes easier to ventilate around the gluteal cleft and makes the diaper 1 less likely to damp.

Furthermore, as shown in FIG. 6A, in the back waist portion 30, of the two openings 50 spaced apart in the lateral direction, the opening 50 located on the lateral one side is defined as a first opening 50a, and the opening 50 located on the lateral other side is defined as a second opening 50b. The first opening 50a and the second opening 50b have a portion where they overlap in the up-down direction, and the distance L5 in the lateral direction from the upper end 50ae of the first opening 50a to the upper end 50be of the second opening 50b is shorter than the length L1, in the up-down direction, of the opening 50. That is, the first opening 50a and the second opening 50b are close to each other both in the up-down and lateral directions. By this arrangement, even if each one opening 50 is small, at least two openings 50 are close to each other, making it easier to visually recognize the openings 50. In addition, by not making the opening 50 excessively large, it becomes difficult for the user's fingers to enter inside when in use, thus reducing the risk of the opening 50 tearing.

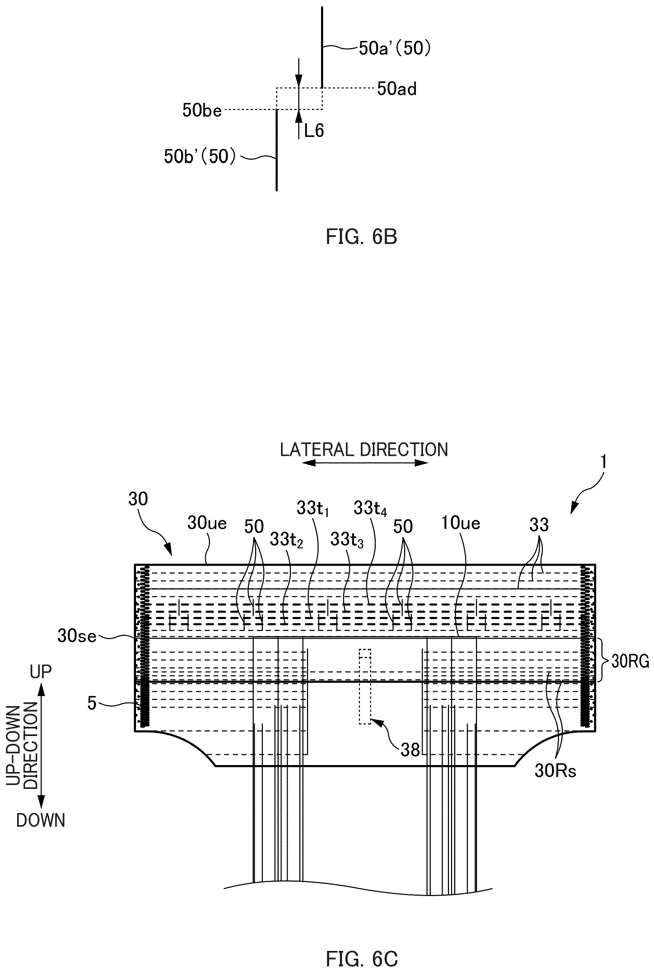

In addition, a first opening 50a and a second opening 50b do not necessarily have to have an overlapping portion in the up-down direction. FIG. 6B is a diagram illustrating a modified example of the arrangement of the first opening 50a and the second opening 50b. As shown in FIG. 6B, the first opening 50a' and the second opening 50b' are provided spaced apart in the up-down direction, and the distance L6 between the first opening 50a' and the second opening 50b', from the lower end 50ad of the upper opening (here, the first opening 50a') to the upper end 50be of the lower opening (here, the second opening 50b'), is shorter than the length L1, in the up-down direction, of the opening 50 (see FIG. 6A). With such a configuration, even if the first opening 50a' and the second opening 50b' are located spaced apart in the up-down direction, the openings 50 are close to each other and even if each opening 50 is small, visually recognition is facilitated.

Also, returning to FIG. 6A, the opening 50 that is adjacent to the first opening 50a in the lateral direction and that is located further on the one side (left side) in the lateral direction relative to the first opening 50a is defined as a third opening 50c. The positional relationship between the first opening 50a and the third opening 50c is the same as the positional relationship between the first opening 50a and the second opening 50b, that is, the second opening 50b and the third opening 50c are formed symmetrically with respect to the first opening 50a. Thus, the first opening 50a, the second opening 50b and the third opening 50c are close to one another and form an opening group 50G. In the back waist portion 30, a plurality of the opening groups 50G are provided spaced apart in the lateral direction, and in the stretched state, the distance L7 in the lateral direction between two opening groups 50G that are adjacent in the lateral direction is longer than the length L1, in the up-down direction, of the opening 50.

By providing the plurality of opening groups 50G, the visibility of the openings 50 can be improved, and also the breathability can be improved. If the distance L7 between the opening groups 50G that are adjacent in the lateral direction is excessively narrow, many discontinuous parts occur in the waist elastic members 33, which may weaken the tightening force of the back waist portion 30. Therefore, by not making the distance L7 between the opening groups 50G excessively close, deterioration of the tightening force can be avoided.

Also, as shown in FIG. 6A, in the stretched state, the first opening 50a and the second opening 50b are separated by a distance R1 in the up-down direction, and it is preferable that the distance R1 be equal to or more than half the length L1, in the up-down direction, of the opening 50. As mentioned above, the distance (L5) in the lateral direction between the first opening 50a and the second opening 50b is narrow, and therefore if the two openings 50a, 50b are not spaced apart in the up-down direction, there is a risk that the openings 50 are less likely to open properly. However, by spacing apart the openings 50 in the up-down direction, the difficulty in opening can be avoided.

In addition, in the present embodiment, a plurality of openings 50 are provided, but in the stretched state, the plurality of openings 50 do not overlap each other in the lateral direction (FIG. 2). If the plurality of openings 50 overlap each other in the lateral direction, when pulling up the diaper 1 at the time of putting on, there is a risk that tears occur between the laterally overlapping openings 50, causing the fit to deteriorate. However, since the openings 50 do not overlap each other in the lateral direction, tearing is less likely to occur. A suitable arrangement of the plurality of openings 50 is to arrange them in a staggered pattern.

As shown in FIGS. 1 and 2, a post-treatment tape 38 which is used to discard an underpants-shaped disposable diaper 1 after use is provided on the non-skin-side face of the back waist portion 30. It is desirable that the openings 50 do not overlap the post-treatment tape 38 in the lateral direction. By ensuring that the openings 50 do not overlap the post-treatment tape 38, it becomes easier to fasten the post-treatment tape 38.

FIG. 6C is a diagram illustrating the waist elastic members 33 that are located in the back waist portion 30 and colored in a specified color. In the present embodiment, it is preferable that the waist elastic members 33 (33t) overlapping the openings 50 in the up-down direction are elastic members colored in a specified color. In FIG. 6C, the dotted lines indicating the colored waist elastic members 33 are shown in thick lines to make the colored waist elastic members 33 easier to be recognized. The waist elastic members 33 (33t) that overlap the openings 50 in the up-down direction are the waist elastic member 33t₁, 33t₂, 33t₃, 33t₄ here, and are shown in thick lines. Further, it is preferable that the specified color be different from the colors of the skin-side sheet 31 and the non-skin-side sheet 32 between which the waist elastic members 33 are sandwiched, thereby making the overlapping waist elastic members 33 more noticeable. The waist elastic members 33t₁, 33t₂, 33t₃, and 33t₄ are colored elastic members, making it easier to draw attention to the colored areas and making it easier to notice the openings 50. This makes the opening 50 more noticeable. In addition, in the process of forming the openings 50 (slits S), by placing colored elastic members in the area where the openings 50 (slits S) are to be formed, there is also the advantage that it is easier to adjust the cutting position during manufacturing.

### Back barrier cuff 30RG

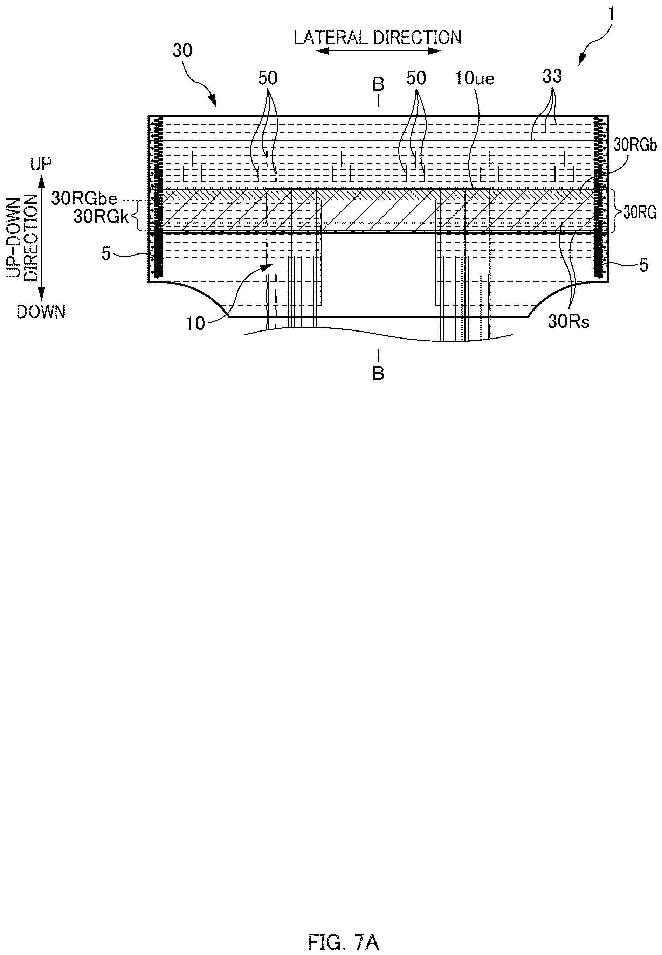

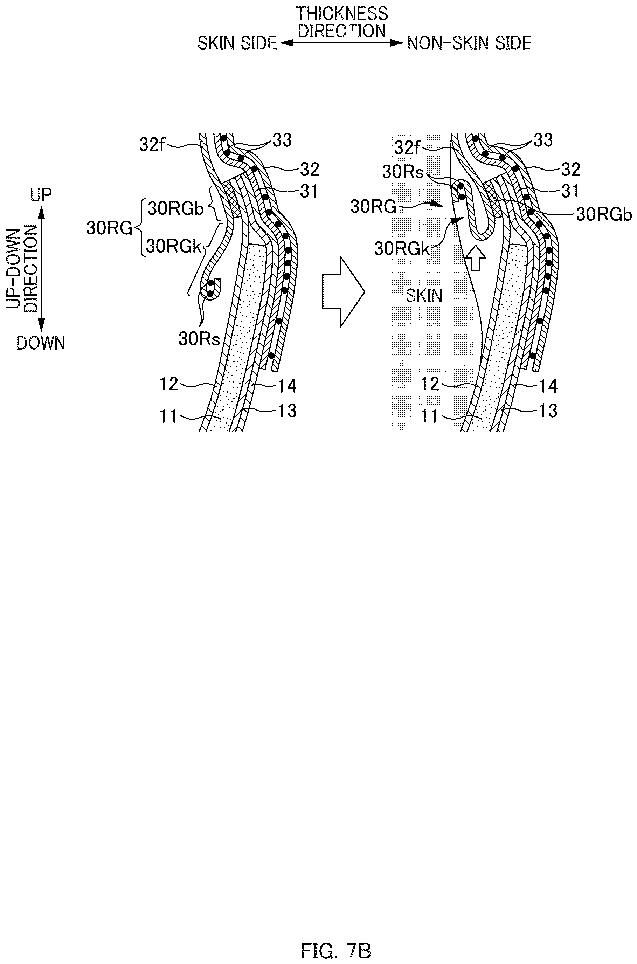

FIG. 7A is a schematic plan view showing the back waist portion 30 in FIG. 2 and illustrating the back barrier cuff 30RG, and FIG. 7B is a diagram illustrating the back barrier cuff 30RG using a cross section of the diaper 1 taken along line B-B in FIG. 7A. As in the present embodiment, in cases where the openings 50 are provided in the back waist portion 30, there is a risk of anxiety that leakage occurs from the openings 50. In this regard, as shown in FIGS. 7A and 7B, the back waist portion 30 includes the back barrier cuff 30RG at the position of the upper end portion of the absorbent main body 10, in the up-down direction. The back barrier cuff 30RG includes: a base end portion 30RGb being incapable of standing up; and a standing portion 30RGk being capable of standing up on the skin side in the thickness direction due to barrier-cuff elastic members 30Rs (elastic strings, etc.), which contract in the lateral direction using the base end portion 30RGb as a fulcrum. In the present embodiment, the back barrier cuff 30RG is formed by the fold-back portion 32f, which is a foldback of the non-skin-side sheet 32, but the back barrier cuff 30RG may also be formed using, for example, a sheet member which is separate from the skin-side sheet 31 and the non-skin-side sheet 32.

The base end portion 30RGb of the back barrier cuff 30RG extends in the lateral direction and is joined and fixed to the top sheet 12. In the standing portion 30RGk, the fold-back portion 32f is further folded back at the end of the standing portion 30RGk toward the non-skin side, with the barrier-cuff elastic members 30Rs, which can stretch/contract in the lateral direction, interposed therebetween. Furthermore, the standing portion 30RGk is located below (on crotch side of) the base end portion 30RGb in the up-down direction; that is, the back barrier cuff 30RG extends from the base end portion 30RGb downward (towards the crotch side). As shown in the right diagram of FIG. 7B, when worn, the contraction of the barrier-cuff elastic members 30Rs causes the standing portion 30RGk to stand up from the top sheet 12, forming the back barrier cuff 30RG. Such standing of the standing portion 30RGk prevents urine, feces, etc. excreted in the crotch portion from spreading to the back waist portion 30. Further, since the base end portion 30RGb is located below the openings 50 in the up-down direction, even with the openings 50 located in the back waist portion 30, the risk of anxiety that leakage occurs can be reduced by blocking the excretion with the standing portion 30RGk. In the present embodiment, two barrier-cuff elastic members 30Rs are used, but the number of the barrier-cuff elastic members 30Rs is not limited to this. Furthermore, without using the barrier-cuff elastic members 30Rs, for example, the back barrier cuff 30RG may be formed using another sheet member having stretchability.

In addition, in the diaper 1, the front waist portion 20 and the back waist portion 30 are joined by the pair of side joining portions 5, 5 in both side portions in the lateral direction, and the back barrier cuff 30RG is also joined by the pair of side joining portions in both side portions in the lateral direction 5, 5 (FIG. 7A). On the other hand, in the back barrier cuff 30RG, the pair of side joining portions 5, 5 are not fixed between the one-side end and the other-side end in the lateral direction. Since, in the back barrier cuff 30RG, the area between the pair of side joining portions 5, 5 is not fixed, the back barrier cuff 30RG can move freely between the pair of side joining portions 5, 5, making it easier for the standing portion 30RGk to stand up. By the putting-on operation, the standing portion 30RGk flips up, and the end portion of the standing portion 30RGk stands up so as to securely touch the skin, creating a pocket-like space and making it less likely to leak (right image of FIG. 7B).

Furthermore, in the present embodiment, the openings 50 are located above the absorbent core 11, and are also located in areas that overlap the absorbent core 11 in the lateral direction, and in areas outside the absorbent core 11, on both sides in the lateral direction. Since the openings 50 are provided not only in the areas that overlap the absorbent core 11 in the lateral direction, but also in the areas outside the absorbent core 11 on both sides in the lateral direction, the stiffness is reduced in the area where the openings 50 exist in the lateral direction, making it more likely to deform. This separates the movement of the upper part of the back waist portion 30 from the movement of the back barrier cuff 30RG, making it easier for the back barrier cuff 30RG to follow the movement of the buttocks and less likely to slip. Therefore, it is possible to improve the fit around the back portion and buttocks portion of the diaper 1.

Also, as shown in FIG. 7A, the lower end 30RGbe, in the up-down direction, of the base end portion 30RGb of the back barrier cuff 30RG is located below the upper end 10ue, in the up-down direction, of the absorbent main body 10. In other words, because the absorbent main body 10 exists below (on the crotch side of) the lower end 30RGbe of the base end portion 30RGb, the discharged liquid blocked by the back barrier cuff 30RG is more likely to be absorbed, making it less likely to leak.

Furthermore, in the diaper 1, the magnitude of the force required to stretch the barrier-cuff elastic member 30Rs by unit length in the lateral direction is smaller than the magnitude of the force required to stretch the waist elastic member 33t, which overlaps the opening 50 in the up-down direction, by unit length in the lateral direction. Since the force required to stretch the barrier-cuff elastic member 30Rs that is placed in the back barrier cuff 30RG is smaller, the back barrier cuff 30RG is more likely to deform, thereby making the diaper 1 easier to fit to the wearer's buttocks.

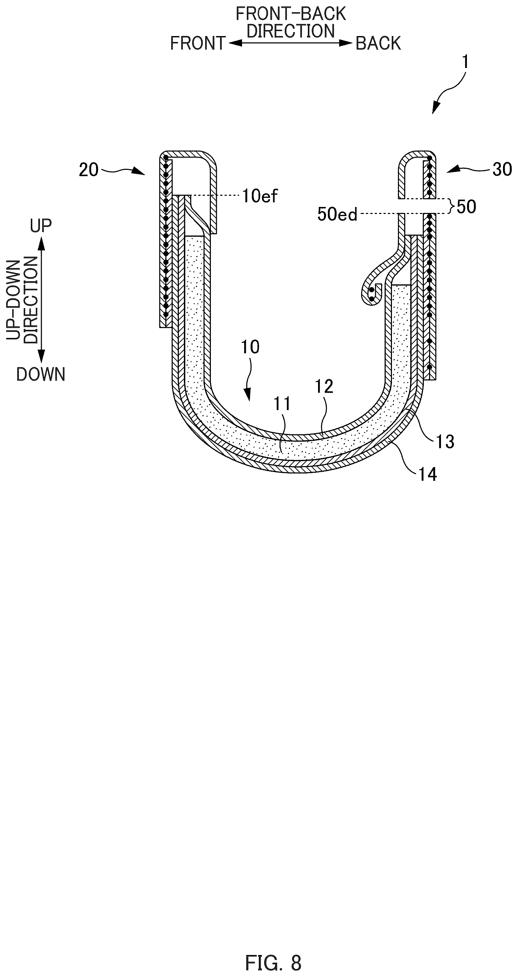

FIG. 8 is a schematic cross-sectional view of the diaper 1 in the underpants-shaped state when being cut in the lateral center of the diaper 1.

As shown in FIG. 8, in the diaper 1 of the present embodiment, the lower ends 50ed, in the up-down direction, of the openings 50 are located below the upper end 10ef, in the up-down direction, of the absorbent main body 10 in the front waist portion 20. On the back waist portion 30 side, the dampness in the wearer's back and gluteal cleft is prevented by placing the openings 50 near the gluteal cleft, whereas, on the front waist portion 20 side, urine leakage is prevented by placing the absorbent main body 10 up to the upper part.

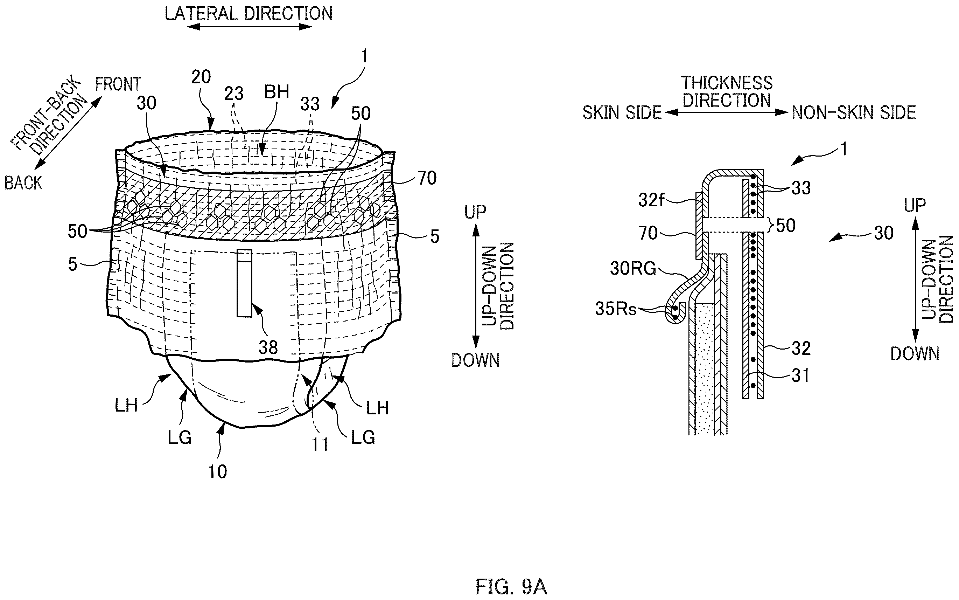

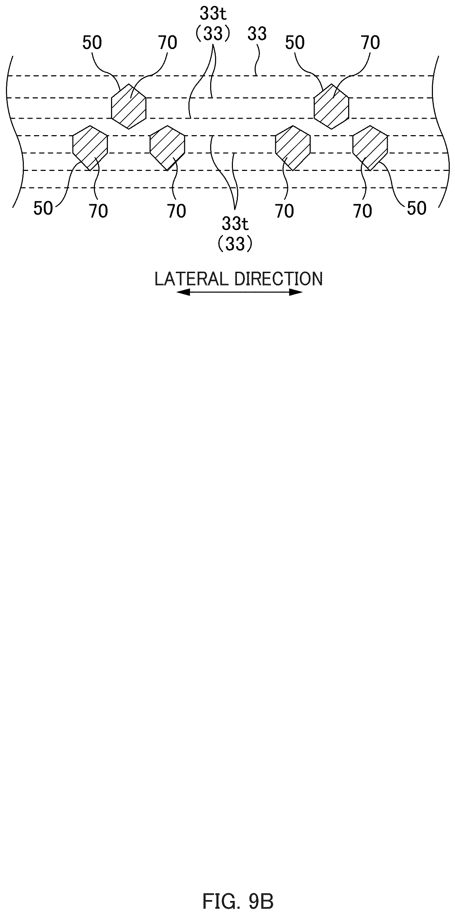

FIG. 9A is a schematic perspective view and schematic cross-sectional view illustrating a modified example in which the back waist portion 30 includes a separated sheet member 70, and FIG. 9B is a diagram illustrating the state of the openings 50 of the diaper 1 of the modified example in the worn state, as viewed from the non-skin side. In the modified example shown in FIG. 9A, the back waist portion 30 of the diaper 1 includes a separated sheet member 70 (indicated by oblique lines in FIG. 9A) which is separate from the skin-side sheet 31 and the non-skin-side sheet 32. The left diagram in FIG. 9A is a diagram of the diaper 1 as viewed from the non-skin side, with the separated sheet member 70 seen through. Specifically, as shown in the right diagram of FIG. 9A, the separated sheet member 70 is located on the skin side of the fold-back portion 32f. And, this separated sheet member 70 covers the openings 50 from the skin side. Here, the separated sheet member 70 extends in the lateral direction between the pair of side joining portions 5, 5, and is large in the up-down direction enough to cover all of the openings 50 from the skin side. But the present invention is not limited to this and the separated sheet member 70 may be large enough to cover any number of the openings 50. In this way, the separated sheet member 70 covers the openings 50 from the skin side, making it possible to reduce the risk of leakage from the openings 50. At the same time, as shown in FIG. 9B, when the diaper 1 is viewed from the non-skin side, the separated sheet member 70 can be seen from inside the openings 50, and it can be seen the openings 50 are blocked by the separated sheet member 70, which also reduces the user's anxiety of leakage.

In the modified example described above, the configuration is not limited to covering the openings 50 with the separated sheet member 70, but it is sufficient that the openings do not penetrate the back waist portion 30 in the thickness direction. Although there are the plurality of openings 50, the fact that the openings 50 do not penetrate the back waist portion 30 can reduce anxiety of leakage.

Furthermore, the above-mentioned separated sheet member 70 may be, for example, a strip-shaped sweat-absorbing sheet that absorbs the wearer's sweat. In cases of the sweat-absorbing sheet, sweat absorbed by the sweat-absorbing sheet evaporates through the openings 50, enabling the diaper 1 to be less likely to damp.

If the separated sheet member 70 is a sweat-absorbing sheet, then the sweat-absorbing sheet 70 may be, for example, laminated nonwoven fabric (not shown) having a three-layered structure. An example of such a laminated nonwoven fabric is one that includes: a first layer located on the skin-facing side of the laminated nonwoven fabric; a second layer located on the non-skin-facing side; and an intermediate layer located between the first layer and the second layer, wherein the first layer and the second layer include hydrophilic fibers and the intermediate layer includes pulp fibers, and wherein the first layer, the second layer, and the intermediate layer are integrated using a known hydroentanglement method such as a water jet. Examples of the above-mentioned hydrophilic fiber include polyethylene terephthalate (PET) fibers, whose fiber surfaces are subject to a hydrophilizing treatment. Both the first layer and the second layer may contain the hydrophilized polyethylene terephthalate (PET) mentioned above, but for the hydrophilic fibers of the second layer, it is preferable to use, for example, rayon fibers having excellent liquid diffusibility, strength after entanglement, versatility, and the like. As for the intermediate layer, it is preferable to use a pulp sheet containing at least pulp fibers. With such a sweat-absorbing sheet 70, sweat adhering to the skin is absorbed by the first layer, then passes through the intermediate layer and quickly moves to the second layer. In cases where the second layer is made of rayon fibers, the excellent diffusibility of the second layer allows sweat to diffuse within the sweat-absorbing sheet 70 and evaporate from the second layer. By having the openings 50 on the non-skin side relative to the sweat-absorbing sheet 70, the absorbed sweat is more likely to evaporate through the openings 50, making the diaper 1 less likely to damp.

Also, the above-mentioned separated sheet member 70 may be nonwoven fabric having openings each of which is smaller than the opening 50. If the nonwoven fabric covers the openings 50 from the skin side, in the parts of the openings 50, the diaper 1 becomes less likely to damp because the openings of the nonwoven fabric are not blocked. Furthermore, as shown in FIG. 9B, when the diaper 1 is viewed from the non-skin side, smaller openings of the nonwoven fabric can be seen through the openings 50, enabling to confirm having good breathability and to give cool impression.

### Modified example 1

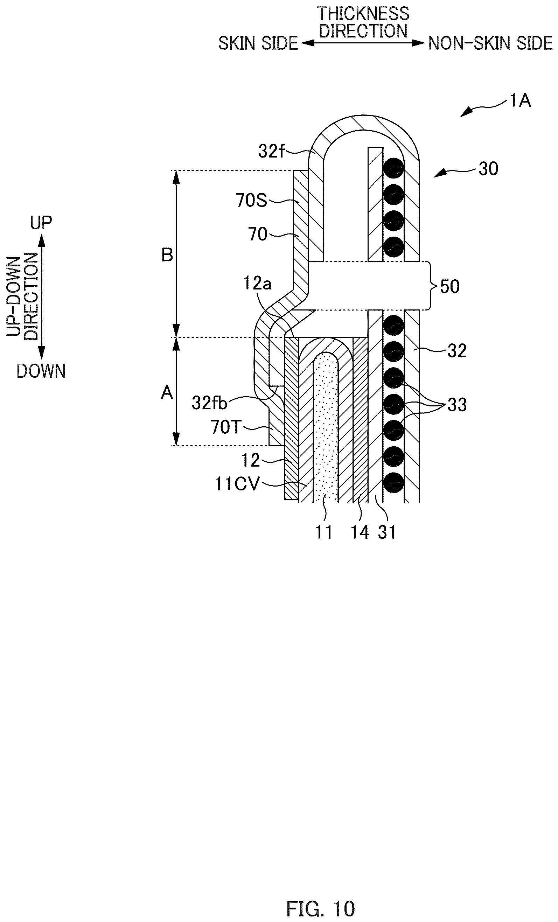

FIG. 10 is a diagram illustrating a modified example 1 of the separated sheet member 70 located in the back waist portion 30. The main difference of a diaper 1A shown in FIG. 10 from the diaper 1 described above is that the back barrier cuff 30RG is not present and the separated sheet member 70 is arranged from the skin side so as to cover a portion where the fold-back portion 32f of the non-skin-side sheet 32 and the top sheet 12 overlap in the thickness direction. Further, FIG. 10 shows a core-wrapping sheet 11CV covering the absorbent core 11. The separated sheet member 70 of the modified example is a sweat-absorbing sheet.

Specifically, as shown in FIG. 10, the sweat-absorbing sheet 70 has an upward extending portion 70S in the back waist portion 30, which extends upward in the up-down direction from the upper end 12a, in the up-down direction, of the top sheet 12. In the diaper 1A, the region where the sweat-absorbing sheet 70 and the top sheet 12 overlap in the thickness direction is defined as an overlapping region A, and the region that is located at the same position in the up-down direction as the upward extending portion 70S of the sweat-absorbing sheet 70 is defined as a region B. Further, in the overlapping region A, the fold-back portion 32f is interposed between the sweat-absorbing sheet 70 and the top sheet 12. In this case, the fold-back portion 32f (part of the non-skin-side sheet 32) is a hydrophobic sheet. The sweat-absorbing sheet 70 has a downward extending portion 70T that extends downward in the up-down direction from the lower end 32fb, in the up-down direction, of the hydrophobic sheet (fold-back portion 32f) (see FIG. 10). The downward extending portion 70T is positioned opposite the top sheet 12 so as to be able to come into contact with it.

Here, the description "hydrophobic" mentioned above means that the liquid does not spread on a sheet surface and the sheet surface has a contact angle greater than 90°. The contact angle is the angle formed between the horizontal plane of a sheet and the tangent to the contour curve of a water droplet, which is dripped onto the horizontal plane, at the intersection point of the contour curve of the water droplet and the horizontal plane, in a state where the sheet is in contact with water droplet. Thus, the above-mentioned hydrophobic sheet means a sheet having a contact angle greater than 90° and a hydrophobic surface.

Thus, the hydrophobic sheet (fold-back portion 32f) is a sheet in which at least the surface is hydrophobic and does not have sweat absorbency, and typical examples include nonwoven fabric containing hydrophobic fibers. In this way, the fold-back portion 32f (hydrophobic sheet), which is impermeable to body fluids such as urine, is interposed between the top sheet 12 and the sweat-absorbing sheet 70, which are arranged on the skin-facing side of the absorbent core 11 in the overlapping region A, thereby effectively preventing liquid from flowing back from the absorbent core 11 to the sweat-absorbing sheet 70.

In addition, in the region B, the sweat-absorbing sheet 70 is arranged from the skin side so as to cover the opening 50, so that sweat absorbed by the sweat-absorbing sheet 70 evaporates through the opening 50, making the back waist portion 30 less likely to damp.

### Modified example 2

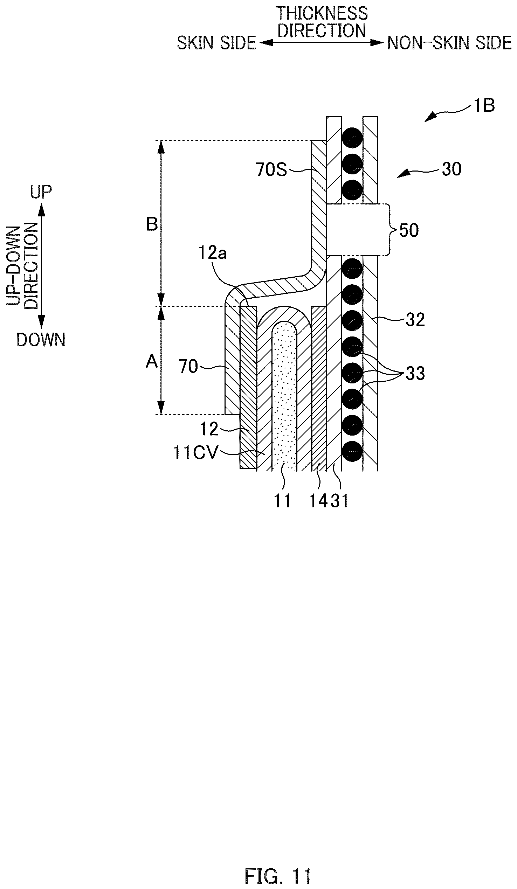

FIG. 11 is a diagram illustrating a modified example 2 of the separated sheet member located in the back waist portion 30. A diaper 1B shown in FIG. 11 is not provided with, like the modified example 1, the back barrier cuff 30RG, but differs from the modified example 1 in that, in the overlapping region A where the sweat-absorbing sheet 70 and the top sheet 12 overlap in the thickness direction, the fold-back portion 32f is not interposed between the sweat-absorbing sheet 70 and the top sheet 12 (therefore, a hydrophobic sheet is not interposed). Since the diaper 1B does not have a hydrophobic sheet (fold-back portion 32f), the diaper 1B is inferior to the diaper 1A which does have it in terms of preventing liquid backflow from the absorbent core 11 to the sweat-absorbing sheet 70. But the other configurations are essentially the same as the modified example 1, and it achieves both smooth sweat transfer from the sweat-absorbing sheet 70 to the top sheet 12 and prevention of liquid backflow. In addition, in the region B, the sweat-absorbing sheet 70 is arranged from the skin side so as to cover the opening 50, so that sweat absorbed by the sweat-absorbing sheet 70 evaporates through the opening 50, making the back waist portion 30 less likely to damp.

### Modified example 3

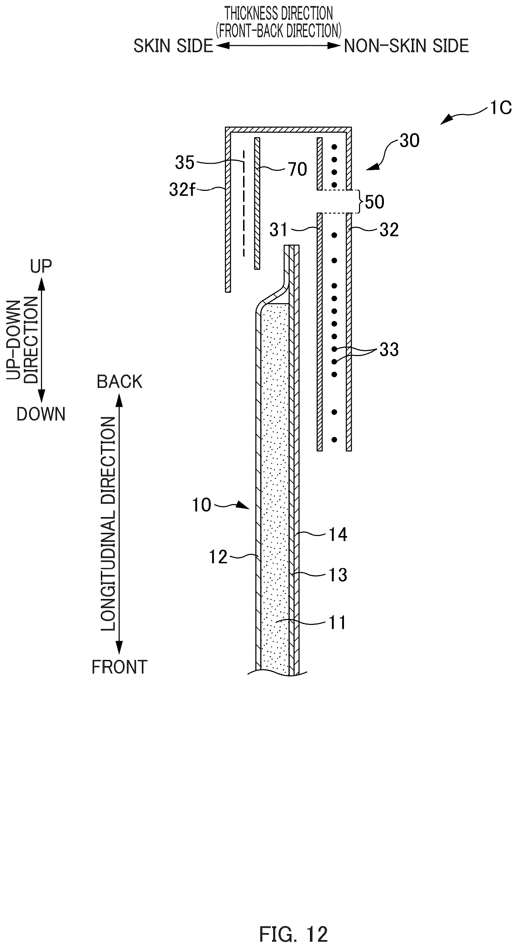

FIG. 12 is a diagram illustrating a modified example 3 of the separated sheet member 70 located in the back waist portion 30. In a diaper 1C shown in FIG. 12, the back barrier cuff 30RG is not provided, as in the modified examples 1 and 2, and the skin-side sheet 31 and the non-skin-side sheet 32 are hydrophobic sheets made of hydrophobic fibers, which allow moisture (such as the wearer's sweat) to pass through. It is desirable that the skin-side sheet 31 and the non-skin-side sheet 32 contain at least one of polyethylene (PE) and polypropylene (PP). Polyethylene and polypropylene have extremely low water absorption ratios, and therefore a sheet containing either of them allows moisture (sweat) to pass through easily.

Also, in the modified example 3, the hydrophilic, separated sheet member 70 (here also referred to as a hydrophilic sheet 70), is arranged on the non-skin side relative to the fold-back portion 32f (the hydrophobic sheet). The hydrophilic sheet 70 is a hydrophilic sheet that can absorb moisture (sweat, etc.). It is desirable that the hydrophilic sheet 70 is a thin paper containing liquid-absorbent pulp fiber (e.g., tissue paper). This improves the sweat absorption of the hydrophilic sheet 70. The definition of hydrophobicity here is the same as above, and a "hydrophilic" sheet means that its surface has the contact angle of less than 90°.

By placing the hydrophilic sheet 70 on the non-skin side of the fold-back portion 32f, the hydrophilic sheet 70 can absorb sweat through the fold-back portion 32f (hydrophobic sheet), and because the fold-back portion 32f is hydrophobic, sweat does not easily return and does not wet the skin. In other words, the surface of the fold-back portion 32f is easily kept dry, and the wearer does not feel sticky, providing a comfortable fit. This improves comfort and reduces the risk of heat rash.

Also, the hydrophilic sheet 70 is located on the skin side relative to the skin-side sheet (hydrophobic sheet) 31. That is, on the non-skin side relative to the hydrophilic sheet 70, two hydrophobic sheets (the skin-side sheet 31 and the unfolded portion of the non-skin-side sheet 32) overlap. This makes it difficult for sweat, etc. absorbed by the hydrophilic sheet 70 to escape outside the back waist portion 30 (diaper 1). Also, the fold-back portion 32f of the non-skin-side sheet 32 and the hydrophilic sheet 70 are joined by a hot-melt adhesive 35. Note that the joining may be done using other joining methods, not just a hot-melt adhesive, but in any method, it is desirable to make joining in an intermittent pattern in the lateral direction or the longitudinal direction (pattern composed of multiple lines or dots). In addition, by placing the hot-melt adhesive 35 intermittently, the passage of sweat from the fold-back portion 32f is not unnecessarily hindered, and the absorbency of the pulp fiber constituting the hydrophilic sheet 70 is not hindered. Furthermore, by placing the openings 50 on the non-skin side relative to the hydrophilic sheet 70, sweat absorbed by the hydrophilic sheet 70 is more easily evaporated from the openings 50, enabling the back waist portion 30 to be less likely to damp.

### Others

The above embodiment of the present disclosure is simply to facilitate understanding of the present disclosure and is not in any way to be construed as limiting the present disclosure. The present disclosure may variously be changed or altered without departing from its gist and encompass equivalents thereof.

In the above-described embodiment, the openings 50 of the back waist portion 30 are located above the absorbent main body 10 in the up-down direction, in the back waist portion 30, but the present invention is not limited to this. For example, the openings 50 may be located in an area that overlaps the absorbent main body 10 in a plan view. The area overlapping the absorbent main body 10 is an area where moisture is more likely to accumulate, and by providing the openings 50 in such an area, it is possible to make it easier for such moisture to escape.

In the above-described embodiment, the distance between the openings 50 adjacent in the lateral direction is short, which are closely spaced, but the present invention is not limited to this. The distance between the openings 50 spaced apart in the lateral direction may be 10 mm or more. If the distance in the lateral direction between the openings 50 is less than 10 mm, the adjacent openings 50 weaken the contractive force acting between them, causing deterioration of the fit and making leakage more likely. However, by setting the distance in the lateral direction between the openings 50 to 10 mm or more, such a decrease in contractive force can be prevented.

### REFERENCE SIGNS LIST

1 diaper (underpants-shaped disposable diaper)
1A diaper (underpants-shaped disposable diaper)
1B diaper (underpants-shaped disposable diaper)
1C diaper (underpants-shaped disposable diaper)
5 side joining portion
10 absorbent main body
11 absorbent core
11CV core-wrapping sheet
12 top sheet
13 back sheet
14 exterior sheet
15 leg elastic member
16 leak-proof wall portion
16r leak-proof-wall elastic member
20 front waist portion
21 skin-side sheet
22 non-skin-side sheet
22f fold-back portion
23 waist elastic member
30 back waist portion
30RG back barrier cuff
30RGb base end portion
30RGk standing portion
30Rs barrier-cuff elastic member
31 skin-side sheet
32 non-skin-side sheet
32f fold-back portion
33 waist elastic member
33ca waist elastic member
33cb waist elastic member
33t waist elastic member
33t₁ overlapping waist elastic member (waist elastic member)
33t₂, 33t₃, waist elastic member
35 hot-melt adhesive
38 post-treatment tape
50 opening
50a, 50a' first opening
50b, 50b' second opening
50c third opening
50G opening group
50h, 50i, 50j, 50k, 50m, 50n opening
70 separated sheet member (sweat-absorbing sheet, hydrophilic sheet)
320 strip-shaped non-skin-side sheet
330 continuous waist elastic member

## Claims

1. An underpants-shaped disposable diaper having an up-down direction, a lateral direction, and a thickness direction that are orthogonal to one another,
the underpants-shaped disposable diaper comprising:
an absorbent main body that has an absorbent core;
a front waist portion;
a back waist portion; and
a plurality of waist elastic members that are arranged spaced apart in the up-down direction and at least in the back waist portion,
the waist elastic members being capable of stretching/contracting in the lateral direction, wherein
the back waist portion has a skin-side sheet and a non-skin-side sheet that is located on a non-skin side relative to the skin-side sheet,
the waist elastic members are provided between the skin-side sheet and the non-skin-side sheet,
the back waist portion has an opening that penetrates the skin-side sheet and the non-skin-side sheet in the thickness direction,
the opening is located at a position that overlaps the absorbent core in the lateral direction and is above the absorbent core in the up-down direction,
in the up-down direction, the opening overlap at least one of the waist elastic members, and
in the lateral direction, the waist elastic member is discontinuous within the opening.

2. An underpants-shaped disposable diaper having an up-down direction, a lateral direction, and a thickness direction that are orthogonal to one another,
the underpants-shaped disposable diaper comprising:
an absorbent main body that has an absorbent core;
a front waist portion;
a back waist portion; and
a plurality of waist elastic members that are arranged spaced apart in the up-down direction and at least in the back waist portion,
the waist elastic members being capable of stretching/contracting in the lateral direction, wherein
the back waist portion has a skin-side sheet and a non-skin-side sheet that is located on a non-skin side relative to the skin-side sheet,
the waist elastic members are provided between the skin-side sheet and the non-skin-side sheet,
the back waist portion has an opening that penetrates the skin-side sheet and the non-skin-side sheet in the thickness direction,
in the up-down direction, the opening overlap at least one of the waist elastic members,
in the lateral direction, the waist elastic member is discontinuous within the opening,
the back waist portion includes a back barrier cuff in an upper end portion, in the up-down direction, of the absorbent main body,
the back barrier cuff has
a base end portion being incapable of standing up, and
a standing portion being capable of standing up on a skin side in the thickness direction due to a barrier-cuff elastic member that contracts in the lateral direction using the base end portion as a fulcrum,
the standing portion is located below the base end portion in the up-down direction, and
the base end portion is located below the opening in the up-down direction.

3. The underpants-shaped disposable diaper according to claim 1 or 2, wherein
a length, in the up-down direction, of the opening is less than 15 mm.

4. The underpants-shaped disposable diaper according to claim 3, wherein
in the stretched state, the opening is a slit extending along the up-down direction.

5. The underpants-shaped disposable diaper according to claim 3, wherein
in the stretched state, the opening is a cutout formed by cutting out a specified range.

6. The underpants-shaped disposable diaper according to claim 1 or 2, wherein
when one of the waist elastic members that overlap the opening in the up-down direction is defined as an overlapping waist elastic member,
in the stretched state, a length, in the up-down direction, of the opening is longer than a distance in the up-down direction between the overlapping waist elastic member and another waist elastic member that is adjacent to the overlapping waist elastic member in the up-down direction.

7. The underpants-shaped disposable diaper according to claim 6, wherein
when the waist elastic member that is located above and adjacent to the overlapping waist elastic member in the up-down direction is defined as an upper waist elastic member, and the waist elastic member that is located below the overlapping waist elastic member is defined as a lower waist elastic member,
in the stretched state, the length, in the up-down direction, of the opening is longer than a distance in the up-down direction from the upper waist elastic member to the lower waist elastic member.

8. The underpants-shaped disposable diaper according to claim 1 or 2, wherein
a magnitude of force required to stretch the waist elastic member that overlaps the opening in the up-down direction, by a unit length in the lateral direction is greater than a magnitude of force required to stretch the waist elastic member that does not overlap the opening in the up-down direction, by the unit length in the lateral direction.

9. The underpants-shaped disposable diaper according to claim 1 or 2, wherein
in the up-down direction, the opening overlaps at least two of the waist elastic members, the at least two waist elastic members being adjacent in the up-down direction, and
in the stretched state,
a distance in the up-down direction between the two waist elastic members adjacent in the up-down direction is shorter than a distance the up-down direction between another two of the waist elastic members,
the other two waist elastic members being adjacent in the up-down direction and not overlapping the opening in the up-down direction.

10. The underpants-shaped disposable diaper according to claim 9, wherein
the plurality of openings that overlap the two waist elastic members adjacent in the up-down direction are provided space apart in the lateral direction.

11. The underpants-shaped disposable diaper according to claim 1 or 2, wherein
in the up-down direction, the opening is provided in a lower region of an area obtained by bisecting between an upper end of the absorbent main body and an upper end of the back waist portion.

12. The underpants-shaped disposable diaper according to claim 1 or 2, wherein
at least two of the openings are provided spaced apart in the lateral direction,
when one of the two openings located on a one side in the lateral direction is defined as a first opening, and the other of the two openings located on another side in the lateral direction is defined as a second opening,
in the stretched state,
a distance in the lateral direction between an upper end of the first opening and an upper end of the second opening is shorter than a length, in the up-down direction, of the opening, and
the first opening and the second opening have an overlapping portion in the up-down direction, or
the first opening and the second opening are spaced apart in the up-down direction, and a distance from a lower end of the upper opening of the first and second openings to an upper end of the lower opening of the first and second openings is shorter than the length, in the up-down direction, of the opening.

13. The underpants-shaped disposable diaper according to claim 12, wherein
the back waist portion has an opening group including the first opening and the second opening,
a plurality of the opening groups are provided spaced apart in the lateral direction, and
in the stretched state, a distance in the lateral direction between two of the opening groups that are adjacent in the lateral direction is longer than the length, in the up-down direction, of the opening.

14. The underpants-shaped disposable diaper according to claim 12, wherein
in the stretched state, the first opening and the second opening are separated in the up-down direction by equal to or more than half the length, in the up-down direction, of the opening.

15. The underpants-shaped disposable diaper according to claim 1 or 2, wherein
a plurality of the openings are provided, and
the plurality of openings do not overlap each other in the lateral direction.

16. The underpants-shaped disposable diaper according to claim 1 or 2, wherein
a post-treatment tape that is used to discard the underpants-shaped disposable diaper after use is provided on a non-skin-side face of the back waist portion, and
the opening does not overlap the post-treatment tape in the lateral direction.

17. The underpants-shaped disposable diaper according to claim 2, wherein
the opening is provided outside the absorbent core on both sides in the lateral direction.

18. The underpants-shaped disposable diaper according to claim 2, wherein
a lower end, in the up-down direction, of the base end portion of the back barrier cuff is located below an upper end, in the up-down direction, of the absorbent main body.

19. The underpants-shaped disposable diaper according to claim 2, wherein
the front waist portion and the back waist portion are joined by a pair of side joining portions in both side portions in the lateral direction,
both side portions, in the lateral direction, of the back barrier cuff are joined by the pair of side joining portions, and
in the back barrier cuff, the pair of side joining portions are not fixed between a one-side end and an other-side end in the lateral direction.

20. The underpants-shaped disposable diaper according to claim 2, wherein
a magnitude of force required to stretch the barrier-cuff elastic member by a unit length in the lateral direction is smaller than a magnitude of force required to stretch the waist elastic member that overlaps the opening in the up-down direction, by the unit length in the lateral direction.

21. The underpants-shaped disposable diaper according to claim 1 or 2, wherein
the opening does not penetrate the back waist portion in the thickness direction.

22. The underpants-shaped disposable diaper according to claim 1 or 2, wherein
the underpants-shaped disposable diaper includes a separated sheet member that is separate from the skin-side sheet and the non-skin-side sheet, and
the separated sheet member covers the opening from a skin side.

23. The underpants-shaped disposable diaper according to claim 22, wherein
the separated sheet member is a sweat-absorbing sheet.

24. The underpants-shaped disposable diaper according to claim 22, wherein
the separated sheet member is nonwoven fabric having an opening that is smaller than the opening.

25. The underpants-shaped disposable diaper according to claim 1 or 2, wherein
a lower end, in the up-down direction, of the opening is located below an upper end, in the up-down direction, of the absorbent main body in the front waist portion.
